(19) Europäisches Patentamt
European Patent Office
Office européen des brevets

(11) EP 4 234 540 A1

(12) EUROPEAN PATENT APPLICATION
published in accordance with Art. 153(4) EPC

(43) Date of publication:
**30.08.2023 Bulletin 2023/35**

(21) Application number: **21882711.1**

(22) Date of filing: **14.10.2021**

(51) International Patent Classification (IPC):
***C07D 257/02*** *(2006.01)* ***A61K 47/60*** *(2017.01)*
***A61K 47/62*** *(2017.01)* ***A61K 47/64*** *(2017.01)*
***A61K 47/68*** *(2017.01)* ***A61K 51/04*** *(2006.01)*

(52) Cooperative Patent Classification (CPC):
**A61K 47/60; A61K 47/62; A61K 47/64; A61K 47/68; A61K 51/04; C07D 257/02**

(86) International application number:
**PCT/JP2021/038135**

(87) International publication number:
**WO 2022/085570 (28.04.2022 Gazette 2022/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.10.2020 JP 2020177566**

(71) Applicant: **Nihon Medi-Physics Co., Ltd**
**Koto-ku**
**Tokyo, 136-0075 (JP)**

(72) Inventors:
- **TAKEMORI, Hideaki**
  **Tokyo 136-0075 (JP)**
- **ICHIKAWA, Hiroaki**
  **Tokyo 136-0075 (JP)**
- **KAWATANI, Minoru**
  **Tokyo 136-0075 (JP)**
- **IZAWA, Akihiro**
  **Tokyo 136-0075 (JP)**
- **IMAI, Tomoyuki**
  **Tokyo 136-0075 (JP)**

(74) Representative: **Zabel, Julia Elisabeth**
**Einsel Attorney at Law und**
**Rechtsanwälte PartmbB**
**Große Bäckerstraße 3**
**20095 Hamburg (DE)**

(54) **METHOD FOR PRODUCING RADIOACTIVE ZIRCONIUM COMPLEX**

(57) The present invention pertains to a method that is for producing a radioactive zirconium labelled complex and that can achieve a high labelling rate in a reaction between a ligand compound and radioactive zirconium ions. The production method according to the present invention comprises a step for causing a reaction between a ligand compound and radioactive zirconium ions in a reaction solution containing water to cause coordination of the radioactive zirconium ions. Said step is performed in a state where the pH of the reaction solution is in an acidic range. The reaction solution does not contain an organic solvent but contains, apart from the ligand compound, a water-soluble organic compound that has, in the structure, one or two sulfo groups or carboxy groups.

## Description

Technical Field

The present invention relates to a method for producing a radioactive zirconium complex.

Background Art

For the purpose of use in reagents and diagnostic agents for detection of target molecules or pharmaceuticals for treatment of diseases, studies on the yield of a radioactive metal complex in which a ligand compound is coordinated to a radioactive metal have been conducted.

Patent Literature 1 discloses that radioactive zirconium and DTPA, which is a kind of ligand compound, were used and that DTPA has been reacted with radioactive zirconium which is a radioactive metal in acidic physiological saline to form a Zr complex.

Patent Literature 2 discloses that radioactive zirconium and DOTA, which is a kind of ligand compound, have been heated and reacted with each other in a neutral HEPES buffer solution to form a Zr complex.

In addition, Non Patent Literature 1 discloses a method in which $^{89}Zr$, which is a radioactive metal, and DOTA, which is a ligand compound, are reacted with each other in a buffer solution to form a radioactive metal complex.

Citation List

Patent Literatures

Patent Literature 1: US 2014/147381 A
Patent Literature 2: US 2019/038785 A

Non Patent Literature

Non Patent Literature 1: Pandya et al, Chem Sci. 2017;8(3):2309-14.

Summary of Invention

However, it has become clear from the findings of the present inventors that under the conditions disclosed in Patent Literatures 1 and 2 and Non Patent Literature 1, complex formation between DOTA and a radioactive zirconium ion does not proceed well, and a sufficient labeling index cannot be achieved in some cases. From such findings, reaction conditions capable of stably achieving a high labeling index have been desired.

Therefore, the present invention is intended to provide a method for producing a radioactive zirconium complex that can stably realize a high labeling index in a reaction between a radioactive zirconium ion and a ligand compound.

The present invention provides a method for producing a radioactive zirconium complex, the method including a step of reacting a radioactive zirconium ion and a ligand compound with each other in a reaction solution containing water to coordinate the radioactive zirconium ion to the ligand compound,

in which the reaction solution contains no organic solvent, and the reaction solution contains the ligand compound and a water-soluble organic compound having one or two sulfo groups or a water-soluble organic compound having one or two carboxy groups, and
in which a pH of the reaction solution is in an acidic region.

Description of Embodiment

Hereinafter, a method for producing a radioactive zirconium complex of the present invention will be described on the basis of a preferable embodiment thereof. The production method of the present invention includes a step (hereinafter also simply referred to as a "step" unless otherwise specified) of reacting a radioactive zirconium ion as a radioactive metal ion and a ligand compound with each other in a reaction solution containing water to coordinate the radioactive zirconium ion to the ligand compound. The present step is performed in a state where the pH of the reaction solution is in an acidic region. Through the present step, a radioactive zirconium complex can be obtained.

This radioactive zirconium complex is a compound in which a radioactive zirconium atom is bonded to the

ligand compound by a combination of a covalent bond, an ionic bond, and the like in addition to a coordinate bond and includes a compound to which a reactive atomic group described later or a targeting agent has been bonded.

In the present specification, coordinating the radioactive zirconium ion to the ligand compound to form a complex and labeling the ligand compound with the radioactive zirconium ion are synonymous, and complexing efficiency and a labeling index are synonymous.

In the following description, unless otherwise specified, "radioactive zirconium" is simply referred to as "radioactive Zr".

From the viewpoint of increasing the labeling index, the radioactive Zr used in the present step is preferably used in the form of a compound capable of being ionized in water, more preferably used in the form of a Zr ion (hereinafter these forms are also collectively referred to as a "radioactive Zr source"). As the radioactive Zr source, for example, a radioactive Zr ion-containing solution in which radioactive Zr ions are dissolved or dispersed in a solvent mainly composed of water can be used.

The nuclide of radioactive Zr is preferably $^{89}Zr$. $^{89}Zr$ is a $\beta^+$-ray decay nuclide and is an electron-capturing decay nuclide. $^{89}Zr$ can be produced, for example, by a nuclear reaction of $^{89}Y(p,n)^{89}Zr$ using a cyclotron. Specifically, a solution obtained by dissolving a $^{89}Y$ target after proton irradiation using an acid is passed through a column cartridge or the like supporting a collector capable of adsorbing $^{89}Zr$. Thereafter, the column cartridge is washed with a solvent such as water, and then an oxalic acid aqueous solution is passed through the column cartridge, so that $^{89}Zr$ ions can be eluted and collected as a solution.

The ligand compound used in the present step is not particularly limited as long as it is a compound capable of being coordinated to radioactive Zr, and examples thereof include the following organic compounds and compounds containing structures derived from the compounds.

CB-TE2A(1,4,8,11-Tetraazabicyclo[6.6.2]hexadecane-4,11-diacetic acid)
CDTA(Cyclohexane-trans-1,2-diamine tetra-acetic acid)
CDTPA(4-cyano-4-[[(dodecylthio)thioxomethyl]thio]-Pentanoic acid)
DOTA(1,4,7,10-Tetraazacyclododecane-1,4,7,10-tetraacetic acid)
DOTMA((1R,4R,7R,10R)-α,α',α",α'"-tetramethyl-1,4,7,10-tetraazacyclododecane-1,4,7,10-tetraacetic acid)
DOTAM(1,4,7,10-tetrakis(carbamoylmethyl)-1,4,7,10-tetraazacyclododecane)
DOTA-GA(α-(2-Carboxyethyl)-1,4,7,10-tetraazacyclododecane-1,4,7,10-tetraacetic acid)
DOTP(((1,4,7,10-Tetraazacyclododecane-1,4,7,10-tetrayl)tetrakis(methylene))tetraphosphonicacid)
DOTMP(1,4,7,10-Tetraazacyclododecane-1,4,7,10-tetrakis(methylenephosphonic acid))
DOTA-4AMP(1,4,7,10-tetraazacyclododecane-1,4,7,10-tetrakis(acetamidomethylenephosphonic acid)
DO2P(Tetraazacyclododecane dimethanephosphonic acid)

Deferoxamine (DFO)

DTPA( N,N-bis[2-[bis(carboxymethyl)amino]ethyl]-glycine)
DTPA-BMA(5,8-Bis(carboxymethyl)-11-[2-(methylamino)-2-oxoethyl]-3-oxo-2,5,8,11-tetraazatridecan-13-oic acid)
EDTA(2,2',2",2'"-(ethane-1,2-diylbis(azanetriyl))tetraacetic acid)
NOTA(1,4,7-Triazacyclononane-1,4,7-triacetic acid)
OTP(1,4,7-Triazacyclononane-1,4,7-triyltris(methylenephosphonic acid)
TETPA(1,4,8,11-tetraazacyclotetradecane-1,4,8,11-tetrapropionic acid)
TETA(1,4,8,11-Tetraazacyclotetradecane-N,N',N",N'"-tetraacetic acid)
TTHA(3,6,9,12-Tetrakis(carboxymethyl)-3,6,9,12-tetraazatetradecanedioic acid)
HEHA(1,2,7,10,13-hexaazacyclooctadecane-1,4,7,10,13,16-hexaacetic acid)
1,2-HOPO(N,N',N",N'"-tetra(1,2-dihydro-1-hydroxy-2-oxopyridine-6-carbonyl)-1,5,10,14-tetraazatetradecane)
PEPA(1,4,7,10,13-pentaazacyclopentadecane-N,N',N",N'",N""-penta-acetic acid)
H4octapa(N,N'-bis(6-carboxy-2-pyridylmethyl)-ethylenediamine-N,N'-diacetic acid)
H2bispa2(6,6'-({9-hydroxy-1,5-bis(methoxycarbonyl)-2,4-di(pyridine-2-yl)-3,7-diazabicyclo[3.3.1]nonane-3,7-diyl}bis(-methylene))dipicolinic acid)
H2dedpa(1,2-[{6-(carboxy)-pyridin-2-yl}-methylamino]ethane)
H2macropa(6-(1,4,10,13-tetraoxa-7,16-diazacyclooctadecan-N,N'-methyl)picolinic acid)
H5decapa(N,N"-bis(6-carboxy-2-pyridylmethyl)-diethylenetriamine-N,N',N"-triacetic acid)
H6phospa(N,N'-(methylenephosphonate)-N,N'-[6-(methoxycarbonyl)pyridin-2-yl]-methyl-1,2-diaminoethane)
HP-D03A(Hydroxypropyltetraazacyclododecanetriaceticacid)

Among these compounds, the ligand compound used in the present step is preferably an organic compound having a structure represented by Formula (1) below.

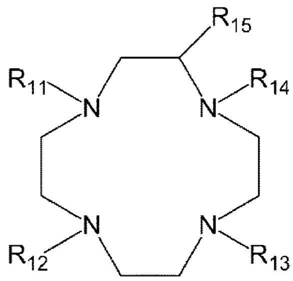

(1)

In Formula (1), $R_{11}$, $R_{12}$, and $R_{13}$ each independently represent a $-(CH_2)_pCOOH$ group, a $-(CH_2)_pC_5H_4N$ group, a $-(CH_2)_pPO_3H_2$ group, or a $-(CH_2)_pCONH_2$ group.
p is each independently an integer of 0 or more and 3 or less.
In Formula (1), one of $R_{14}$ and $R_{15}$ is a hydrogen atom, a $-(CH_2)_pCOOH$ group, a - $(CH_2)_pC_5H_4N$ group, a $-(CH_2)_pPO_3H_2$ group, a $-(CH_2)_pCONH_2$, group or a - $(CHCOOH)(CH_2)_pCOOH$ group.
In Formula (1), the other one of $R_{14}$ and $R_{15}$ is a $-(CH_2)_pCOOH$ group, a - $(CH_2)_pC_5H_4N$ group, a $-(CH_2)_pPO_3H_2$ group, a $-(CH_2)_pCONH_2$ group, a reactive atomic group to be linked to the targeting agent or a group linked to the targeting agent.
p is each independently an integer of 0 or more and 3 or less.
Details of the targeting agent and the reactive atomic group to be linked to the targeting agent or the group linked to the targeting agent will be described later.
The ligand compound used in the present step is more preferably a compound containing a structure derived from 1,4,7,10-tetraazacyclododecane-1,4,7,10-tetraacetic acid (DOTA) or a derivative thereof, and specifically, it is more preferable to include one of the compounds shown below or a structure derived from the compound. The ligand compound used in the present step is preferably water-soluble.

DOTA(1,4,7, 10-Tetraazacyclododecane-1,4,7,10-tetraacetic acid)
DOTMA((1R,4R,7R,10R)-α,α',α'',α'''-tetramethyl-1,4,7,10-tetraazacyclododecane-1,4,7,10-tetraacetic acid)
DOTAM(1,4,7,10-tetrakis(carbamoylmethyl)-1,4,7,10-tetraazacyclododecane)
DOTA-GA(α-(2-Carboxyethyl)-1,4,7,10-tetraazacyclododecane-1,4,7,10-tetraacetic acid)
DOTP(((1,4,7,10-Tetraazacyclododecane-1,4,7,10-tetrayl)tetrakis(methylene))tetraphosphonicacid)
DOTMP(1,4,7,10-Tetraazacyclododecane-1,4,7,10-tetrakis(methylenephosphonic acid))
DOTA-4AMP(1,4,7,10-tetraazacyclododecane-1,4,7,10-tetrakis(acetamidomethylenephosphonic acid)
DO2P(Tetraazacyclododecane dimethanephosphonic acid)

The reaction solution in the present step is an aqueous reaction solution containing water and a water-soluble organic compound having a predetermined structure. The reaction solution does not contain an organic solvent.
The above-described water-soluble organic compound is an organic compound that dissolves in water and is a compound different from the above-described ligand compound and the organic solvent described later. Therefore, the water-soluble organic compound in the present specification is not included in the ligand compound or the organic solvent. The water-soluble organic compound used in the present step is not particularly limited as long as it has one or two sulfo groups or one or two carboxy groups.
In the following description, a water-soluble organic compound that has a predetermined structure and is not included in the ligand compound or the organic solvent is also referred to as a "second organic compound".
As the water, water commonly used in the present technical field can be employed, and for example, distilled water or ion-exchanged water can be used.
The reaction solution used in the present step contains no organic solvent. Examples of the organic solvent that is not contained include water-soluble organic solvents such as polar solvents such as protic solvents such as methanol and ethanol, and aprotic solvent such as acetonitrile, N,N-dimethylformamide, tetrahydrofuran, dimethyl sulfoxide, and acetone; and water-insoluble organic solvents such as hexane, toluene, and ethyl acetate. Not including such an organic solvent eliminates the need for a step of separating the organic solvent, such as solid phase extraction, and thus a high labeling index can be stably achieved while avoiding radiolysis by concentration.
The phrase "not contain an organic solvent" means that an organic solvent is not intentionally contained in the reaction solution, but inevitable mixing of an organic solvent in the reaction solution is acceptable.
The second organic compound contained in the reaction solution is a compound that exhibits a pH buffering

action in a predetermined pH range when the compound is dissolved in water but does not necessarily exhibit a buffering action in the reaction solution of the present step. That is, the reaction solution itself containing the second organic compound used in the present step may not exhibit a pH buffering action or may exhibit a pH buffering action depending on the pH of the entire reaction solution.

One of the characteristics of the second organic compound contained in the reaction solution is that the second organic compound has a specific functional group in the structure thereof. Specifically, as an embodiment of the second organic compound, the second organic compound has one or two sulfo groups in its structure. The sulfo group is a monovalent functional group represented by "-$SO_3H$" or "-$SO_3^-$". That is, the second organic compound in the present embodiment may be a monosulfonic acid or a disulfonic acid.

In the case where sulfo group(s) is included in the structure, the total carbon number of the second organic compound is preferably 4 or more and 10 or less, more preferably 6 or more and 8 or less.

In the case where sulfo group(s) is included in the structure, the second organic compound preferably has a hetero atom in the structure, preferably has at least a nitrogen atom in the structure, further preferably has a cyclohexane ring or a heterocyclic ring in the structure, still more preferably has two nitrogen atoms or contains a saturated heterocyclic ring having a nitrogen atom and an oxygen atom in the structure, and still more preferably contains a morpholine ring or a piperazine ring in the structure.

In the case where sulfo group(s) is included in the structure, it is also preferable that an alkanesulfonic acid group be included in the structure, it is also preferable that the alkanesulfonic acid group be bonded to a hetero atom, and it is more preferable that an aminoalkanesulfonic acid is included in the structure.

In any of the cases described above, the second organic compound having sulfo group(s) in its structure is preferably a zwitterionic compound, more preferably an aminoalkanesulfonic acid derivative.

Examples of the second organic compound having one sulfo group in its structure include chain amine monosulfonic acids such as N-tris(hydroxymethyl)methyl-2-aminoethanesulfonic acid (TES); monosulfonic acids having a morpholine ring, such as 2-morpholinoethanesulfonic acid (MES) and 3-morpholinopropanesulfonic acid (MOPS); and/or salts thereof.

Examples of the second organic compound having two sulfo groups in its structure include a disulfonic acid having a piperazine ring in the structure, such as 2-[4-(2-hydroxyethyl)-1-piperazinyl]ethanesulfonic acid (HEPES) and piperazine-1,4-bis(2-ethanesulfonic acid) (PIPES); and/or salts thereof.

Examples of the counter ion of the second organic compound having sulfo group(s) in its structure include ions of alkali metals such as sodium and potassium, cations such as primary to quaternary ammonium such as ammonium and a tetramethylammonium salt, and anions of halogens and the like such as chlorine.

As another embodiment of the second organic compound contained in the reaction solution, one or two carboxy groups are included in the structure. The carboxy group is a monovalent functional group represented by "-COOH" or "-$COO^-$". That is, the second organic compound in the present embodiment may be a monocarboxylic acid or a dicarboxylic acid.

In the case where carboxy group(s) is included in the structure, the total carbon number of the second organic compound is preferably 2 or more and 10 or less, more preferably 2 or more and 8 or less.

In the case where carboxy group(s) is included in the structure, the second organic compound is preferably a saturated or unsaturated aliphatic carboxylic acid or aromatic carboxylic acid, more preferably a saturated aliphatic carboxylic acid.

Examples of the second organic compound having one carboxy group in its structure include linear aliphatic dicarboxylic acids such as acetic acid and lactic acid; aromatic monocarboxylic acids such as benzoic acid and salicylic acid; and/or salts thereof.

Examples of the second organic compound having two carboxy groups in its structure include linear aliphatic monocarboxylic acids such as malonic acid and tartaric acid; aromatic dicarboxylic acids such as phthalic acid; and/or salts thereof.

Examples of the counter ion of the second organic compound having carboxy group(s) in its structure include ions of alkali metals such as sodium and potassium, cations such as primary to quaternary ammonium such as ammonium and a tetramethylammonium salt, and anions of halogens and the like such as chlorine.

Among these, as the second organic compound having sulfo group(s) or carboxy group(s) in its structure, it is more preferable to use one of acetic acid, phthalic acid, and malonic acid, or 2-[4-(2-hydroxyethyl)-1-piperazinyl]ethanesulfonic acid, N-tris(hydroxymethyl)methyl-2-aminoethanesulfonic acid, and 2-morpholinoethanesulfonic acid, and it is still more preferable to use acetic acid or 2-[4-(2-hydroxyethyl)-1-piperazinyl]ethanesulfonic acid.

Since such an organic compound is relatively easily available, the labeling index can be further increased while reducing the production cost. The reaction solution containing a suitable second organic compound can be used in the present step in a state of being prepared in advance as an aqueous solution containing these organic compounds. These may be in the form of buffer solutions that exhibit a pH buffering action or in the form of liquids that do not exhibit a pH buffering action in the present step.

From the viewpoint of further increasing the labeling efficiency, the concentration of the second organic compound in the reaction solution is preferably 0.01 mol/L or more and 2.0 mol/L or less, more preferably 0.1 mol/L or more and 1.0 mol/L or less.

In the present step, the order of addition of the radioactive Zr source and the ligand compound is not limited as long as the labeling reaction of the ligand compound with the radioactive Zr ion can proceed, specifically, the complex formation between the radioactive Zr ion and the ligand compound can be performed. For example, one of the radioactive Zr source and the ligand compound may be added to a reaction vessel already accommodating a mixed solvent containing water and the second organic compound constituting the reaction solution and not containing an organic solvent, and then the other may be added and reacted. Alternatively, one of the radioactive Zr source and the ligand compound may be added to a solution obtained by dissolving the other in a mixed solvent to cause the reaction. Alternatively, the radioactive Zr source and the ligand compound may be simultaneously added to a reaction vessel already accommodating a mixed solvent to cause the reaction.

In the present step, the reaction in the reaction solution is performed in a state where the pH of the reaction solution is in the acidic region. In the present step, the reaction is carried out in a state where the acidic state of the pH is maintained from the start to the end of the reaction. The fact that the pH of the reaction solution is in the acidic region means that the pH of the reaction solution during that step is less than 7.

From the viewpoint of reducing the interaction with radioactive Zr ions that may adversely affect the radiolabeling and further increasing the labeling efficiency, the present step is performed in a state where the pH of the reaction solution is preferably 2.0 or more and 6.0 or less.

As described above, the reaction solution containing the second organic compound used in the present step may be a buffer solution that exhibits a pH buffering action from the start to the end of the reaction or may be a liquid that does not exhibit a pH buffering action.

The pH of the reaction solution is adjusted in advance so as to be in the acidic region before the reaction is started, that is, before the present step is performed, whereby the pH of the reaction solution can be maintained in the acidic region even during the present step.

The pH of the reaction solution can be adjusted, for example, by mixing an aqueous solution of the second organic compound whose pH has been adjusted in advance into the reaction solution. In addition, the pH of the reaction solution can be adjusted by preparing each of a radioactive Zr ion-containing solution, an aqueous solution of the ligand compound, and an aqueous solution of the second organic compound in advance and mixing these aqueous solutions at an adjusted mixing ratio. Alternatively, the pH of the reaction solution can be adjusted by adding an inorganic acid such as hydrochloric acid or a metal hydroxide such as sodium hydroxide to a liquid in which the radioactive Zr ion, the ligand compound, and the second organic compound are mixed.

The reaction conditions in the present step can be, for example, the following conditions.

As the reaction solution used in the present step, an aqueous liquid containing water and the second organic compound and not containing an organic solvent is used.

The pH of the reaction solution used in the present step is adjusted to an acidic region.

The reaction pressure can be atmospheric pressure.

In the present step, from the viewpoint of achieving further improvement in labeling efficiency in a short production time, it is preferable to carry out the reaction by heating the reaction solution. The heating means applying heat from the outside of the reaction system such that the temperature of the reaction solution becomes higher than 25°C on the basis of 25°C. As a method for applying heat from the outside of the reaction system, a known method can be appropriately used, and examples thereof include a water bath, an oil bath, a block heater, and a heating mantle.

In the case where the reaction solution is heated to carry out the reaction, the reaction solution is heated to a reaction temperature of preferably 30°C or higher and 100°C or lower, more preferably 50°C or higher and 80°C or lower, from the viewpoint of achieving both suppression of the decomposition of the ligand compound and further improvement of the labeling efficiency.

The reaction time is preferably 15 minutes or more and 150 minutes or less, more preferably 30 minutes or more and 120 minutes or less, on condition that the reaction temperature is as described above.

The amount of the reaction solution in the present step may be appropriately changed according to the production scale but is practically 0.01 mL or more and 100 mL or less at the start of the present step from the viewpoint of practicality in the production step.

From the viewpoint of increasing the yield of the target radioactive Zr-labeled compound, the concentrations of the radioactive Zr ions and the ligand compound in the reaction solution are each independently preferably 1 μmol/L or more and 1,000 μmol/L or less, more preferably 10 μmol/L or more and 900 μmol/L or less, still more preferably 30 μmol/L or more and 600 μmol/L or less, still more preferably 50 μmol/L or more and 500 μmol/L or less at the start of the present step.

In the production method of the present invention including the step described above, since radioactive Zr and the ligand compound are easily dissolved in the reaction solution, the labeling reaction can uniformly proceed in the

liquid phase. In addition to this, in the present step, the reaction is performed in a state where the water-soluble organic compound having a predetermined structure is present in the reaction system and in a state kept in the acidic region, whereby the labeling index can be further increased as compared with the prior art, and a target radioactive Zr complex can be stably generated. In addition, since the yield of the obtained radioactive Zr complex is high, it is also advantageous in that the complex can be subjected to subsequent steps without separating and purifying unreacted radioactive Zr.

The present inventors presume as follows the reason why the labeling index can be stably improved by carrying out the reaction in a state where the second organic compound having a predetermined structure is present in the reaction system and in a state kept in the acidic condition.

In the reaction solution maintained in the acidic region, the ligand compound maintains a chemical structure capable of reacting with the radioactive Zr ion, and the radioactive Zr ion also retains an oxidation state that enables the reaction with the ligand compound. On the other hand, the second organic compound having a predetermined structure has a chemical structure in the reaction solution that does not have an adverse effect such as unintentional coordination to a radioactive Zr ion in the acidic region or has a reaction rate at which the adverse effect occurs sufficiently slower than a reaction rate with the ligand compound, which allows the labeling reaction, which is the main reaction, to advantageously proceed. As a result, it is presumed that the reaction between the radioactive Zr ion and the ligand compound proceeds, and the labeling index can be stably increased.

As shown in examples described later, since the second organic compound having a predetermined structure causes the labeling reaction to proceed well even under pH conditions greatly deviating from the pH range in which the pH buffering action is exhibited, it is presumed that the stable progress of the labeling reaction in the present invention is not caused by exhibition of the pH buffering action in the liquid potentially possessed by the water-soluble organic compound. In this regard, as shown in examples described later, the present inventors have confirmed that the labeling index is inferior to that of the production method of the present invention in the case where an acidic physiological saline not containing the second organic compound is used as the reaction solution, which is the conditions disclosed in Patent Literature 1. Therefore, it is also presumed that the second organic compound in the reaction solution plays a role of favorably advancing the labeling reaction between the radioactive Zr ion and the ligand compound by maintaining the pH of the reaction solution in the acidic region.

From the viewpoint of improving both the handleability of the ligand compound to be used and the stability of the resulting radioactive Zr complex, particularly the stability of complex formation, each of $R_{11}$, $R_{12}$, and $R_{13}$ is preferably a carboxyalkyl group represented by a $-(CH_2)_pCOOH$ group, where p is an integer of 1 or more and 3 or less.

In this case, it is also preferable that one of $R_{14}$ and $R_{15}$ be a hydrogen atom or a carboxyalkyl group represented by a $-(CH_2)_pCOOH$ group, where p is an integer of 1 or more and 3 or less. In this case, it is also preferable that the other one of $R_{14}$ and $R_{15}$ is a carboxyalkyl group represented by a $-(CH_2)_pCOOH$ group, where p is an integer of 1 or more and 3 or less, or a reactive atomic group to be linked to the targeting agent or a linked group.

In the case where $R_{11}$, $R_{12}$, $R_{13}$, $R_{14}$, and $R_{15}$ have the above-described suitable groups and where one of $R_{14}$ and $R_{15}$ is a hydrogen atom, the other of $R_{14}$ and $R_{15}$ is preferably a reactive atomic group to be linked to the targeting agent or a group linked to the targeting agent.

That is, in the case where $R_{14}$ represents a reactive atomic group to be linked to the targeting agent or a group linked to the targeting agent, $R_{15}$ represents preferably a hydrogen atom, and in the case where $R_{15}$ represents a reactive atomic group to be linked to the targeting agent or a group linked to the targeting agent, $R_{14}$ represents preferably a hydrogen atom.

In the case where a ligand compound containing a group linked to the targeting agent in Formula (1) is used, the targeting agent is preferably one kind or two or more kinds among atomic groups including those selected from the group consisting of chain peptides, cyclic peptides, or combinations thereof, proteins, antibodies or fragments thereof, peptide aptamers, growth factors, Affibody, UniBody, Nanobody, monosaccharides, polysaccharides, vitamins, antisense nucleic acids, siRNAs, miRNAs, nucleic acid aptamers, decoy nucleic acids, cPG oligonucleic acids, peptide nucleic acids, liposomes, micelles, carbon nanotubes, and nanoparticles.

The "targeting agent" in the present specification refers to a chemical structure that gives rise to directionality to a target organ or tissue in a living body or specificity to a target molecule. In the present specification, a target organ or tissue and a target molecule are also collectively referred to as a "target site".

These targeting agents may be directly bonded to the ligand compound or indirectly bonded through other known linker structures such as PEG.

In addition, these targeting agents may be configured to be capable of being linked to the ligand compound using a modified reactive atomic group that can be bonded to another structure. In order to achieve the linkage to the ligand compound, for example, a known reaction such as a click reaction can be employed.

In the case where a click reaction is used for linking, for example, both the reactive atomic group of the targeting agent and the reactive atomic group of the ligand compound to be linked to the targeting agent can be groups containing click-reactive atomic groups.

By using the ligand compound having such a chemical structure, it is possible to easily achieve bonding to the

targeting agent having specificity or directionality to a target site, and it is possible to obtain the radioactive Zr complex having specificity or directionality to the target site with high yield in a state where the specificity or directionality to the target site of the targeting agent is sufficiently maintained.

In the case where the targeting agent includes a peptide, the atomic group preferably includes a chain peptide, a cyclic peptide, or a combination thereof, a protein, or an antibody or a fragment thereof that is specifically bonded to a specific molecule.

Examples of such an atomic group include peptides having three or more constituent amino acid residues, such as antibodies (immunoglobulins) of the IgG, IgA, IgM, IgD, and IgE classes, antibody fragments such as Fab fragments and F(ab')2 fragments, and peptide aptamers. In addition, an amino acid constituting such a targeting agent may be natural or synthetic.

The molecular weight of the above atomic group including a peptide is not particularly limited.

Various peptides that can be used as the targeting agent can be synthesized by conventionally known methods, such as techniques such as a liquid phase synthesis method, a solid phase synthesis method, an automated peptide synthesis method, a gene recombination method, a phage display method, genetic code reprogramming, and a random non-standard peptide integrated discovery (RaPID) method. In the synthesis of various peptides, functional groups of amino acids to be used may be protected as necessary.

In the case where the targeting agent is an atomic group containing a nucleic acid, the atomic group is preferably an atomic group containing an antisense nucleic acid, siRNA, miRNA, nucleic acid aptamer, decoy nucleic acid, cPG oligo-nucleic acid, or peptide nucleic acid that is specifically bonded to a specific molecule. In addition, the nucleobase constituting such a targeting agent may be natural ones, such as a deoxyribonucleic acid and a ribonucleic acid, or synthetic ones.

The atomic group containing the above-described nucleic acid that can be used in the present invention can be produced by a conventionally known method. For example, in the case of a nucleic acid aptamer, a nucleic acid aptamer that is specifically bonded to a specific target substance such as a protein can be produced using systematic evolution of ligands by exponential enrichment (SELEX).

In the case where a ligand compound containing a click-reactive atomic group is used as the ligand compound containing a reactive atomic group to be linked to the targeting agent used in the present invention, a click-reactive atomic group derived from a known reagent that can be used for a click reaction can be appropriately used.

The "reactive atomic group" in the present specification refers to a chemical structure that directly undergoes a reaction for bonding one compound to the other compound. Examples of such a reactive atomic group include, but are not limited to, click-reactive atomic groups.

From the viewpoint of simplifying the reaction process, the click-reactive atomic group as the reactive atomic group is preferably an atomic group that can be used for a metal-catalyst-free click reaction. Examples of such a structure include an alkynyl group, an azide group, and a diene or a dienophile such as 1,2,4,5-tetrazine and an alkenyl group.

The click reaction is a reaction of a combination of an alkyne and an azide or a combination of a diene and a dienophile, such as 1,2,4,5-tetrazine and an alkene. Specific examples of the click reaction of such a combination of atomic groups include a Huisgen cycloaddition reaction and an inverse electron demand Diels-Alder reaction.

Typically, the chemical structure produced by the click reaction of the combination of an alkyne and an azide contains a triazole skeleton, and the chemical structure produced by the click reaction of the combination of 1,2,4,5-tetrazine and an alkene as the combination of a diene and a dienophile contains a pyridazine skeleton. Therefore, a triazole skeleton can be formed by a click reaction in the case where the click-reactive atomic group that can be contained in the reactive atomic group to be linked to the targeting agent is an atomic group containing an alkyne or an azide. Alternatively, a pyridazine skeleton can be formed by a click reaction in the case where the click-reactive atomic group that can be contained in the reactive atomic group to be linked to the targeting agent is an atomic group containing 1,2,4,5-tetrazine or an alkene as a diene or a dienophile.

Specific examples of the click-reactive atomic group include an atomic group containing dibenzylcyclooctyne (DBCO) as an alkyne (Formula (5a)), an atomic group containing an azide group as an azide (Formula (5b)), an atomic group containing 1,2,4,5-tetrazine (Formula (5c)), and an atomic group containing trans-cyclooctene (TCO) as an alkene (Formula (5d)) as shown in the following formulas.

(5a) 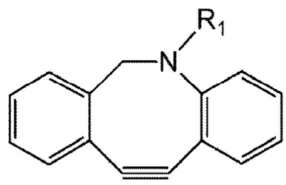

Dibenzylcyclooctyne

(5b) 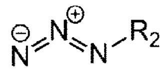

Azide

(5c) 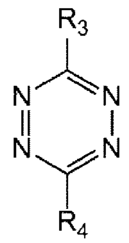

1,2,4,5-tetrazine

(5d) 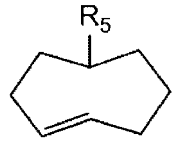

*trans*-cyclooctene

In Formula (5a), $R_1$ represents a bonding site to an atomic group including the ligand compound or the targeting agent.

In Formula (5b), $R_2$ represents a bonding site to an atomic group including the ligand compound or the targeting agent.

In Formula (5c), one of $R_3$ and $R_4$ represents a bonding site to an atomic group including the ligand compound or the targeting agent, and the other represents a hydrogen atom, a methyl group, a phenyl group, or a pyridyl group.

In Formula (5d), $R_5$ represents a bonding site to an atomic group including the ligand compound or the targeting agent.

In the case where a click-reactive atomic group is introduced into the ligand compound, it can be introduced using various commercially available reagents. Specifically, in the case where an atomic group containing dibenzylcyclooctyne (DBCO) is introduced as the click-reactive atomic group, for example, DBCO reagents such as DBCO-C6-Acid, DBCO-Amine, DBCO-Maleimide, DBCO-PEG acid, DBCO-PEG-NHS ester, DBCO-PEG-Alcohol, DBCO-PEG-amine, DBCO-PEG-NH-Boc, Carboxyrhodamine-PEG-DBCO, Sulforhodamine-PEG-DBCO, TAMRA-PEG-DBCO, DBCO-PEG-Biotin, DBCO-PEG-DBCO, DBCO-PEG-Maleimide, TCO-PEG-DBCO, and DBCO-mPEG can be used.

In the case where the structure of Formula (1) is used as the ligand compound, for example, a ligand compound having a structure represented by any of following Formulas (1-a) to (1-e) can be used as a suitable ligand compound used in the present invention, but the ligand compound is not limited thereto. Even with the ligand compound having any structure, the effect of stably improving the labeling index is sufficiently exhibited. In each of the following formulas, P represents an atomic group containing a reactive atomic group or an atomic group containing the targeting agent. From the viewpoint of stably improving the labeling index, a ligand compound having a structure represented by above Formula (1-b), (1-d), or (1-e) is more preferably used.

(1-a)

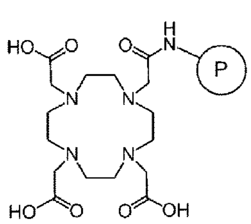

(1-b)

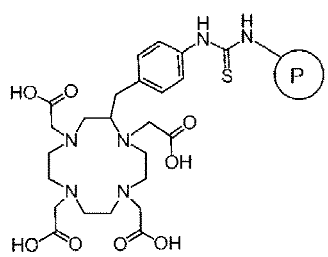

(1-c)

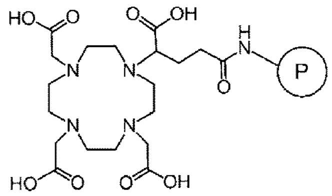

(1-d)

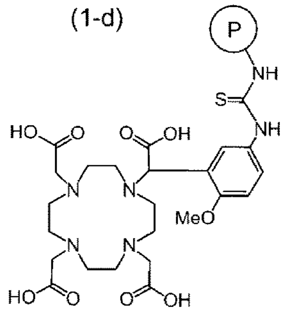

(1-e)

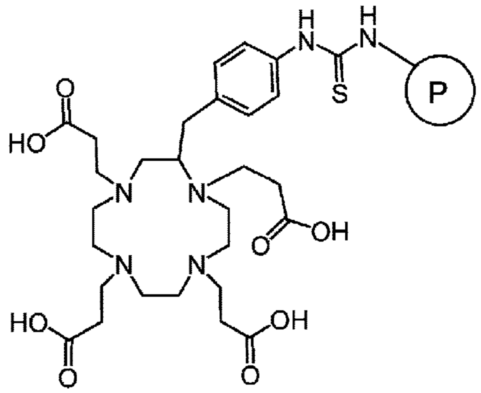

In the case where a ligand compound having the structure of Formula (1) and containing a reactive atomic group is used as the ligand compound, the reactive atomic group is preferably a click-reactive atomic group, and it is also preferable that the ligand compound and the click-reactive atomic group are indirectly bonded by a linker structure represented by following Formula (P). The structure is a structure derived from ethylene glycol, and in Formula (P), n is preferably an integer of 2 or more and 10 or less, more preferably an integer of 2 or more and 8 or less.

(P) 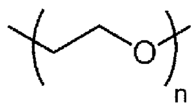

The structure of the ligand compound containing a click-reactive atomic group is not particularly limited as long as the effect of the present invention is exhibited, but it is more preferable to have the following structure. That is, the ligand compound more preferably contains at least one of DO3A-DBCO, DOTA-DBCO, DO3A-PEG4-DBCO, DO4A-PEG7-Tz, and DOTAGA-DBCO shown below.

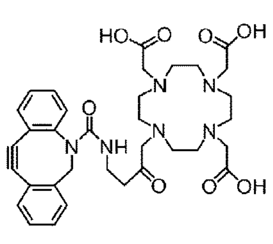

DO3A-DBCO

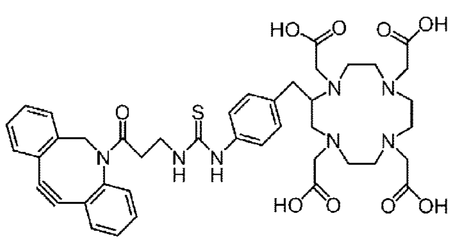

DOTA-DBCO

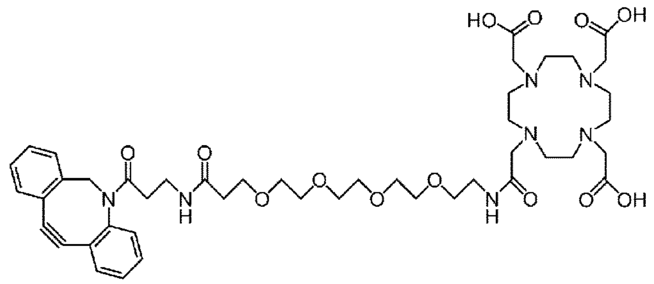

DO3A-PEG4-DBCO

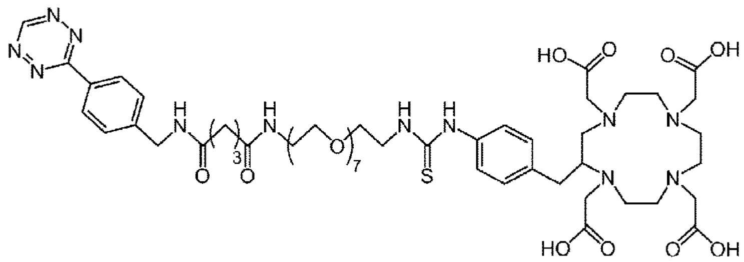

DO4A-PEG7-Tz

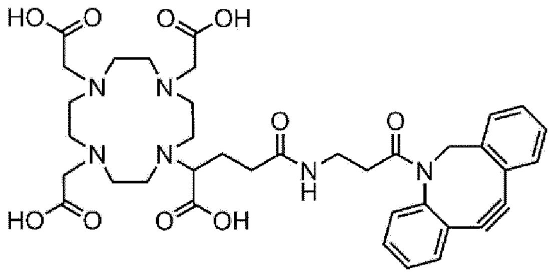

(X)

DOTAGA-DBCO

In the case where a ligand compound containing a click-reactive atomic group as the reactive atomic group is used, for example, a radioactive Zr ion is coordinated to the ligand compound by the above-described method, and then a click reaction or the like of the click-reactive atomic group of the ligand compound to which the radioactive Zr ion has been coordinated and the click-reactive atomic group of the targeting agent is carried out, whereby a radioactive Zr complex can be produced.

In this case, as the targeting agent, a compound modified with a click-reactive atomic group that is specifically bonded to a reactive atomic group in the ligand compound can be used.

As the click-reactive atomic group for modifying the targeting agent, the same group as those described above can be used. By using such a compound, a radioactive Zr complex having specificity or directionality to the target site can be produced.

The radioactive Zr complex generated through the above-described steps exists in a state of being dissolved in the reaction solution. That is, the radioactive Zr complex can be obtained as an aqueous liquid. The aqueous liquid containing the radioactive Zr complex may be used as it is or may be purified using a filtration filter, a membrane filter, a column filled with various fillers, chromatography, or the like.

Examples of the step after the radioactive Zr complex is obtained include a formulation step for obtaining a radioactive drug containing the radioactive Zr complex as an active ingredient. The formulation step can be performed by appropriately adding various additives such as a pH adjusting agent such as a citrate buffer solution, a phosphate buffer solution, and a borate buffer solution, a solubilizing agent such as polysorbate, a stabilizer, and an antioxidant, or by adjusting the radioactivity concentration by dilution with water or an isotonic solution such as physiological saline. In addition, the formulation step may include a step of adding various additives or adjusting the concentration and then performing sterilizing filtration with a membrane filter or the like to prepare an injection.

Examples

Hereinafter, the present invention will be described in more detail with reference to examples. However, the scope of the present invention is not limited to such examples. The following examples were all carried out at atmospheric pressure. In the following description, unless otherwise specified, the pH at the time of performing the step is simply referred to as "pH", and a field indicated by "-" in the following table indicates not performed.

Examples 1 to 4

$^{89}Zr$ was used as the radioactive Zr element. DOTA (in Formula (1) above, $R_{11}$, $R_{12}$, $R_{13}$, and $R_{14}$ are all "-$CH_2COOH$" groups, and $R_{15}$ is a hydrogen atom) was used as the ligand compound.

The above ligand compound was dissolved in water for injection to prepare an aqueous solution containing 20 mmol/L of the above ligand compound (Examples 1 to 3) or an aqueous solution containing 0.2 mmol/L of the above ligand compound (Example 4).

As for Examples 1 to 3, in a reaction vessel (glass vial), 1.5 μL of the aqueous solution of the ligand compound, 100 μL of a $^{89}Zr$ ion-containing solution (solvent: 0.1 mol/L hydrochloric acid aqueous solution, radioactivity concentration: 1.57 to 6.51 MBq/mL) as the radioactive Zr source, and 198.5 μL of a 0.1 mol/L acetic acid-sodium acetate aqueous solution (pH: 4.0 to 6.0) as the second organic compound were mixed to prepare each of reaction solutions having various concentrations and pH values shown in Table 1 below. All of these reaction solutions did not contain an organic solvent.

As for Example 4, a reaction solution having various concentrations and pH values shown in Table 1 below was prepared by mixing in the same manner as in Example 1 except that 150 μL of the aqueous solution of the ligand compound and 50 μL of a 0.78 mol/L acetic acid-sodium acetate aqueous solution (pH: 6.0) as the second organic compound were added. This reaction solution did not contain an organic solvent.

Then, while the pH of each reaction solution was maintained, the reaction solution was heated at a heating temperature and a heating time shown in Table 1 below to provide a solution containing a $^{89}Zr$ complex as a radioactive Zr-labeled compound.

Using thin-layer chromatography (manufactured by Merck & Co., Inc., model number: RP-18, developing solvent: 10 vol% ammonium chloride aqueous solution/methanol (1 : 1)), 2 μL of the obtained $^{89}Zr$ complex solution was developed at a development distance of 10 cm.

The thin-layer chromatogram after development was introduced into a TLC analyzer (GITA Star), and the total $^{89}Zr$ radioactivity count including unreacted $^{89}Zr$ and the radioactivity count of the $^{89}Zr$ complex in the $^{89}Zr$ complex solution were each measured. Then, the percentage of the radioactivity count of the $^{89}Zr$ complex relative to the total $^{89}Zr$ radioactivity count was calculated as the labeling index (%). The labeling index indicates the degree of progress of the labeling reaction, and a higher labeling index means that a larger amount of the target radioactive Zr-labeled compound is generated and that the labeling reaction is progressing well. The results are shown in Table 1 below.

Separately from this, the radioactivity count remaining in the reaction vessel was measured, and the percentage of the remaining radioactivity count with respect to the total radioactivity count (radioactivity prepared) in the reaction solution was calculated as the adsorption ratio (%). In addition, the radiochemical yield (%; hereinafter also referred to as RCY) was calculated using following Calculation Formula (I). The smaller the adsorption ratio is and the higher the RCY is, the more the target radioactive Zr-labeled complex can be used in the subsequent steps, which means that the productivity is improved. The results are shown in Table 1 below.

$$\text{Radiochemical yield (\%)} = \text{labeling index} \times (100 - \text{adsorption ratio})/100 \quad \ldots \text{(I)}$$

Examples 5 and 6 and Comparative Examples 1 and 2

A 0.1 mol/L HEPES-NaOH aqueous solution (pH: 6.0 to 8.0) was used as the second organic compound in place of the acetic acid-sodium acetate aqueous solution to prepare respective reaction solutions having various concentrations and pH values shown in Table 1 below. All of these reaction solutions did not contain an organic solvent. The operation and evaluation were performed in the same manner as in Example 1 except for this point. The results are shown in Table 1 below.

Example 7

A 0.1 mol/L sodium phthalate aqueous solution (pH: 4.0) was used as the second organic compound in place of the acetic acid-sodium acetate aqueous solution to prepare a reaction solution having various concentrations and pH

values shown in Table 1 below. This reaction solution did not contain an organic solvent. Then, the heating time of the reaction solution was changed as shown in Table 1 below. The operation and evaluation were performed in the same manner as in Example 1 except for this point. The results are shown in Table 1 below.

Example 8

A 0.1 mol/L sodium malonate aqueous solution (pH: 4.0) was used as the second organic compound in place of the acetic acid-sodium acetate aqueous solution to prepare a reaction solution having various concentrations and pH values shown in Table 1 below. This reaction solution did not contain an organic solvent. Then, the heating time of the reaction solution was changed as shown in Table 1 below. The operation and evaluation were performed in the same manner as in Example 1 except for this point. The results are shown in Table 1 below.

Example 9

A 0.1 mol/L MES aqueous solution (pH: 4.0) was used as the second organic compound in place of the acetic acid-sodium acetate aqueous solution to prepare a reaction solution having various concentrations and pH values shown in Table 1 below. This reaction solution did not contain an organic solvent. Then, the heating time of the reaction solution was changed as shown in Table 1 below. The operation and evaluation were performed in the same manner as in Example 1 except for this point. The results are shown in Table 1 below.

Example 10

A 0.1 mol/L TES aqueous solution (pH: 4.0) was used as the second organic compound in place of the acetic acid-sodium acetate aqueous solution to prepare a reaction solution having various concentrations and pH values shown in Table 1 below. This reaction solution did not contain an organic solvent. Then, the heating time of the reaction solution was changed as shown in Table 1 below. The operation and evaluation were performed in the same manner as in Example 1 except for this point. The results are shown in Table 1 below.

Example 11

DOTAGA-DBCO (Formula (X) above) was used as the ligand compound in place of DOTA.

The ligand compound was dissolved in a 0.1 mol/L acetic acid-sodium acetate aqueous solution (pH: 5.0) to prepare a solution containing 300 mmol/L of the ligand compound.

As for Example 11, in a reaction vessel (glass vial), 150 μL of the aqueous solution of the ligand compound, 100 μL of a $^{89}Zr$ ion-containing solution (solvent: 0.1 mol/L hydrochloric acid solution, radioactivity concentration: 62 MBq/mL) as the radioactive Zr source, and 100 μL of a 0.1 mol/L acetic acid-sodium acetate aqueous solution (pH: 5.0) as the second organic compound were mixed to prepare a reaction solution having various concentrations and pH values shown in Table 1 below. This reaction solution did not contain an organic solvent. The operation and evaluation were performed in the same manner as in Example 1 except for this point. The results are shown in Table 1 below.

Comparative Examples 3 and 4

A 0.1 mol/L Tris-HCl aqueous solution (pH: 8.0 to 9.0) was used as the second organic compound in place of the acetic acid-sodium acetate aqueous solution to prepare reaction solutions having various concentrations and pH values shown in Table 1 below. This reaction solution did not contain an organic solvent. The operation and evaluation were performed in the same manner as in Example 1 except for this point. The results are shown in Table 1 below.

Comparative Example 5

A0.1 mol/L sodium citrate aqueous solution (pH: 4.5) was used as the second organic compound in place of the acetic acid-sodium acetate aqueous solution to prepare a reaction solution having various concentrations and pH values shown in Table 1 below. This reaction solution did not contain an organic solvent. The operation and evaluation were performed in the same manner as in Example 1 except for this point. The results are shown in Table 1 below.

Comparative Example 6

Physiological saline (0.154 mol/L sodium chloride aqueous solution) was used as the second organic compound

in place of the acetic acid-sodium acetate aqueous solution to prepare a reaction solution having various concentrations and pH values shown in Table 1 below. This reaction solution did not contain an organic solvent. Then, the reaction was performed without heating while the reaction solution was maintained at room temperature (25°C). The operation and evaluation were performed in the same manner as in Example 1 except for this point. The results are shown in Table 1 below.

Comparative Example 7

Physiological saline (0.154 mol/L sodium chloride aqueous solution) was used as the second organic compound in place of the acetic acid-sodium acetate aqueous solution to prepare a reaction solution having the concentration and pH shown in Table 1 below. This reaction solution did not contain an organic solvent. The operation and evaluation were performed in the same manner as in Example 1 except for this point. The results are shown in Table 1 below.

[Table 1]

| | Composition of reaction solution | | | | | | | Heating condition | | Result of labeling with radioactive Zr | | |
|---|---|---|---|---|---|---|---|---|---|---|---|---|
| | Zr radioactivity concentration [MBq/L] | Zr ion concentration [pmol/L] | Second organic compound | | Ligand compound | | pH | Temperature [°C] | Time [min] | Labeling index [%] | Adsorption ratio [%] | RCY [%] |
| | | | Type | Concentration [mol/L] | Type | Concentratio n [$\mu$mol/L] | | | | | | |
| Ex. 1 | 2133 | 1443 | Acetic acid-Na acetate | 0.07 | DOTA | 100 | 4.0 | 70 | 30 | 93 | 9 | 85 |
| Ex. 2 | 2143 | 1450 | Acetic acid-Na acetate | 0.07 | DOTA | 100 | 4.7 | 70 | 30 | 92 | 16 | 77 |
| Ex. 3 | 523 | 354 | Acetic acid-Na acetate | 0.07 | DOTA | 100 | 5.8 | 70 | 30 | 79 | 57 | 34 |
| Ex. 4 | 2170 | 1468 | Acetic acid-Na acetate | 0.13 | DOTA | 100 | 4.7 | 70 | 30 | 83 | 27 | 61 |
| Ex. 5 | 2157 | 1459 | HEPES | 0.07 | DOTA | 100 | 2.0 | 70 | 30 | 93 | 9 | 84 |
| Ex. 6 | 2163 | 1463 | HEPES | 0.07 | DOTA | 100 | 3.0 | 70 | 30 | 96 | 8 | 89 |
| Comp. Ex. 1 | 513 | 347 | HEPES | 0.07 | DOTA | 100 | 7.0 | 70 | 30 | 0 | 40 | 0 |
| Comp. Ex. 2 | 2143 | 1450 | HEPES | 0.07 | DOTA | 100 | 8.0 | 70 | 30 | 8 | 39 | 5 |
| Ex. 7 | 2507 | 1695 | Na phthalate | 0.07 | DOTA | 100 | 4.0 | 70 | 60 | 83 | - | - |
| Ex. 8 | 2627 | 1777 | Na malonate | 0.07 | DOTA | 100 | 4.0 | 70 | 60 | 80 | - | - |
| Ex. 9 | 2630 | 1779 | MES | 0.07 | DOTA | 100 | 4.0 | 70 | 60 | 83 | - | - |
| Ex. 10 | 2497 | 1689 | TES | 0.07 | DOTA | 100 | 4.0 | 70 | 60 | 94 | - | - |
| Ex. 11 | 17714 | 11980 | Acetic acid-Na acetate | 0.07 | DOTAGA-DBCO | 129 | 4.0 | 70 | 30 | 89 | 27 | 65 |
| Comp. Ex. 3 | 2120 | 1434 | Tris | 0.07 | DOTA | 100 | 2.0 | 70 | 30 | 50 | 16 | 42 |
| Comp. Ex. 4 | 2150 | 1454 | Tris | 0.07 | DOTA | 100 | 8.5 | 70 | 30 | 58 | 78 | 13 |

(continued)

| | Composition of reaction solution | | | | | | | Heating condition | | Result of labeling with radioactive Zr | | |
|---|---|---|---|---|---|---|---|---|---|---|---|---|
| | Zr radioactivity concentration [MBq/L] | Zr ion concentration [pmol/L] | Second organic compound | | Ligand compound | | pH | Temperature [°C] | Time [min] | Labeling index [%] | Adsorption ratio [%] | RCY [%] |
| | | | Type | Concentration [mol/L] | Type | Concentratio n [μmol/L] | | | | | | |
| Comp. Ex. 5 | 1007 | 681 | Na citrate | 0.07 | DOTA | 100 | 4.5 | 70 | 30 | 10 | - | - |
| Comp. Ex. 6 | 9167 | 6200 | NaCl | 0.154 | DOTA | 100 | 1.0 | 25 | 30 | 6 | - | - |
| Comp. Ex. 7 | 9067 | 6133 | NaCl | 0.154 | DOTA | 100 | 1.0 | 70 | 30 | 53 | - | - |

As described above, it is found that in the production method of the examples in which the second organic compound having a predetermined structure is used and in which the reaction solution having a pH maintained in the acidic region is heated to cause the reaction, the labeling reaction proceeds well, and a high labeling index can be achieved in the reaction of Zr ions with a ligand compound containing DOTA or a DOTA derivative, as compared with the production method of the comparative examples.
In addition, in Examples 1, 2, 5, and 6 in which the reaction solution having a pH maintained in the suitable acidic region was heated, high RCY can be achieved with less adsorption to the reaction vessel while achieving a high labeling index. As a result, the productivity of the Zr complex can be further increased.
In addition, comparison between Example 2 and Example 4 shows that both the labeling index and RCY can be further improved in the example in which the concentration of the second organic compound is lower.
As described above, the present invention provides a production method that can realize a high labeling index in a reaction of a radioactive zirconium ion with a ligand compound.

## Claims

1. A method for producing a radioactive zirconium complex, the method comprising: a step of reacting a radioactive zirconium ion and a ligand compound with each other in a reaction solution containing water to coordinate the radioactive zirconium ion to the ligand compound,

   wherein the reaction solution contains no organic solvent,
   the reaction solution contains:

      the ligand compound; and
      a water-soluble organic compound having one or two sulfo groups or a water-soluble organic compound having one or two carboxy groups, and

   a pH of the reaction solution is in an acidic region.

2. The method for producing a radioactive zirconium complex according to claim 1, wherein the pH of the reaction solution is 2.0 or more and 6.0 or less.

3. The method for producing a radioactive zirconium complex according to claim 1 or 2, wherein a concentration of the water-soluble organic compound contained in the reaction solution is 0.01 mol/L or more and 2.0 mol/L or less.

4. The method for producing a radioactive zirconium complex according to any one of claims 1 to 3, wherein the water-soluble organic compound includes acetic acid, phthalic acid, or malonic acid, or 2-[4-(2-hydroxyethyl)-1-piperazinyl]ethanesulfonic acid, N-tris(hydroxymethyl)methyl-2-aminoethanesulfonic acid, or 2-morpholinoethanesulfonic acid.

5. The method for producing a radioactive zirconium complex according to any one of claims 1 to 4, wherein the reaction solution is heated to 30°C or higher and 100°C or lower to react the radioactive zirconium ion with the ligand compound.

6. The method for producing a radioactive zirconium complex according to any one of claims 1 to 5, wherein the ligand compound is represented by Formula (1) below:

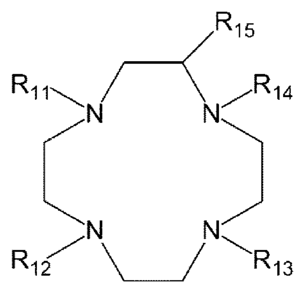

(1)

   wherein $R_{11}$, $R_{12}$, and $R_{13}$ each independently represent a $-(CH_2)_pCOOH$ group, a$-(CH_2)_pC_5H_4N$ group, a

$-(CH_2)_pPO_3H_2$ group, or a $-(CH_2)_pCONH_2$ group,
one of $R_{14}$ and $R_{15}$ represents a hydrogen atom, a $-(CH_2)_pCOOH$ group, a - $(CH_2)_pC_5H_4N$ group, a $-(CH_2)_pPO_3H_2$ group, a $-(CH_2)_pCONH_2$ group, or a - $(CHCOOH)(CH_2)_pCOOH$ group, the other one represents a $-(CH_2)_pCOOH$ group, a - $(CH_2)_pC_5H_4N$ group, a $-(CH_2)_pPO_3H_2$ group, a $-(CH_2)_pCONH_2$ group, a reactive atomic group to be linked to a targeting agent, or a group linked to the targeting agent, and p is each independently an integer of 0 or more and 3 or less.

**7.** The method for producing a radioactive zirconium complex according to claim 6,

wherein in the formula, all of $R_{11}$, $R_{12}$, and $R_{13}$ represent $-(CH_2)_pCOOH$ groups,
one of $R_{14}$ and $R_{15}$ represents a hydrogen atom or a $-(CH_2)_pCOOH$ group,
the other of $R_{14}$ and $R_{15}$ represents a $-(CH_2)_pCOOH$ group, the reactive atomic group to be linked to the targeting agent or the group linked to the targeting agent,
$R_{15}$ represents a hydrogen atom in a case where $R_{14}$ represents the reactive atomic group to be linked to the targeting agent or the group linked to the targeting agent, and
$R_{14}$ represents a hydrogen atom in a case where $R_{15}$ represents the reactive atomic group to be linked to the targeting agent or the group linked to the targeting agent.

**8.** The method for producing a radioactive zirconium complex according to claim 6 or 7, wherein the targeting agent includes an atomic group containing one or two or more selected from the group consisting of a chain peptide, a cyclic peptide, or a combination thereof, a protein, an antibody or a fragment thereof, a growth factor, Affibody, UniBody, Nanobody, a monosaccharide, a polysaccharide, a vitamin, an antisense nucleic acid, a siRNA, a miRNA, a nucleic acid aptamer, a decoy nucleic acid, a cPG oligonucleic acid, a peptide nucleic acid, a liposome, a micelle, a carbon nanotube, and a nanoparticle.

**9.** The method for producing a radioactive zirconium complex according to claim 6 or 7, wherein the reactive atomic group to be linked to the targeting agent includes an azido group, an alkynyl group, a diene or a dienophile.

**10.** The method for producing a radioactive zirconium complex according to any one of claims 6 to 9,

wherein the radioactive zirconium ion is coordinated to the ligand compound using the ligand compound having the reactive atomic group to be linked to the targeting agent, and then
the reactive atomic group is reacted with the targeting agent.

**INTERNATIONAL SEARCH REPORT**

International application No. **PCT/JP2021/038135**

**A. CLASSIFICATION OF SUBJECT MATTER**

***C07D 257/02***(2006.01)i; ***A61K 47/60***(2017.01)i; ***A61K 47/62***(2017.01)i; ***A61K 47/64***(2017.01)i; ***A61K 47/68***(2017.01)i; ***A61K 51/04***(2006.01)i
FI: C07D257/02; A61K47/62; A61K47/64; A61K47/68; A61K47/60; A61K51/04 200; A61K51/04 100

According to International Patent Classification (IPC) or to both national classification and IPC

**B. FIELDS SEARCHED**

Minimum documentation searched (classification system followed by classification symbols)

C07D257/02; A61K47/60; A61K47/62; A61K47/64; A61K47/68; A61K51/04

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2019/126982 A1 (HENKEL AG & CO. KGAA) 04 July 2019 (2019-07-04) claims, p. 1, lines 14-18, p. 17, line 12 to p. 18, line 18, p. 46, lines 17-29, p. 47, lines 1-18, p. 49, line 18 to p. 50, line 15 | 1-10 |
| X | WO 2017/161356 A1 (WAKE FOREST UNIVERSITY) 21 September 2017 (2017-09-21) claims, p. 1, lines 8-16, p. 12, scheme, p. 41, lines 6-10, table 6, schemes 3, 4 | 1-10 |
| X | WO 2015/046278 A1 (FUJIFILM CORP) 02 April 2015 (2015-04-02) claims, paragraphs [0007]-[0009], [0046], [0246]-[0248], examples 29-32, text examples | 1-10 |
| A | PARKER, D. et al. Journal of the Chemical Society. Dalton Transactions. Inorganic Chemistry. 1994, pp. 689-693 entire text, all drawings | 1-10 |
| P, X | WO 2021/142258 A1 (FUSION PHARMACEUTICALS INC.) 15 July 2021 (2021-07-15) claims, p. 2, lines 1-4, examples 25-27, 30, 35-36 | 1-10 |

☐ Further documents are listed in the continuation of Box C.

☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 December 2021** | **21 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/038135**

| Patent document cited in search report | Publication date (day/month/year) | Patent family member(s) | Publication date (day/month/year) |
|---|---|---|---|
| WO 2019/126982 A1 | 04 July 2019 | US 2020/0317970 A1<br>EP 3732262 A1<br>KR 10-2020-0093686 A<br>CN 111511864 A | |
| WO 2017/161356 A1 | 21 September 2017 | US 2019/0038785 A1 | |
| WO 2015/046278 A1 | 02 April 2015 | US 2016/0199520 A1<br>claims, paragraphs [0021]-[0024], [0055], [0212]-[0214], examples 29-32, text examples<br>EP 3050878 A<br>CA 2961935 A<br>IL 251500 D | |
| WO 2021/142258 A1 | 15 July 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2014147381 A
- US 2019038785 A


### Non-patent literature cited in the description


- **PANDYA et al.** *Chem Sci.,* 2017, vol. 8 (3), 2309-14